(19) Europäisches Patentamt / European Patent Office / Office européen des brevets

(11) **EP 2 603 183 B1**

(12) **EUROPEAN PATENT SPECIFICATION**

(45) Date of publication and mention of the grant of the patent:
**28.03.2018 Bulletin 2018/13**

(21) Application number: **11746013.9**

(22) Date of filing: **12.08.2011**

(51) Int Cl.:
***A61F 7/00*** ^(2006.01)

(86) International application number:
**PCT/GB2011/051530**

(87) International publication number:
**WO 2012/020267 (16.02.2012 Gazette 2012/07)**

(54) **A BODY PART TEMPERATURE REGULATING APPARATUS**

VORRICHTUNG ZUR REGELUNG DER TEMPERATUR EINES KÖRPERTEILS

APPAREIL DE RÉGULATION DE LA TEMPÉRATURE D'UNE PARTIE DE L'ORGANISME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.08.2010 GB 201013631**

(43) Date of publication of application:
**19.06.2013 Bulletin 2013/25**

(73) Proprietor: **Paxman Coolers Limited**
**Huddersfield HD8 0LE (GB)**

(72) Inventors:
• **PAXMAN, Glenn**
  **Huddersfield HD9 7DN (GB)**
• **BURKE, Patrick**
  **Sheffield S10 5FN (GB)**

(74) Representative: **Appleyard Lees IP LLP**
**15 Clare Road**
**Halifax HX1 2HY (GB)**

(56) References cited:
**WO-A1-99/44552      WO-A2-2008/114218**
**US-A1- 2004 210 283      US-A1- 2008 077 202**
**US-A1- 2010 152 824**

**EP 2 603 183 B1**

**Description**

**BACKGROUND**

**Technical Field**

The invention relates to a body part temperature regulating apparatus for regulating the temperature of a part of the human or animal body. In particular, but not exclusively, the invention relates to a scalp cooling apparatus. In particular, but not exclusively, the invention relates to a scalp cooling apparatus for regulating the temperature of the scalp of a human being undergoing chemotherapy, or other such-like treatment.

**Description of Related Art**

The benefits of cooling the scalp during chemotherapy treatment have been known for some time. United States Patent 4,566,455 (Kramer) was published in 1986 and discloses an improved scalp cover which provides for the circulation of liquid coolant to reduce and control scalp temperature during chemotherapy. According to this document, scalp cooling during chemotherapy can alleviate hair loss by reducing metabolic activity in the scalp.

International patent publication WO 98/16176 (Olofsson) discusses the problem of hair loss suffered by patients undergoing chemotherapy in more detail. According to this document, cooling the skin, and therewith the roots of the hair, to a temperature of from 0 °C to +5 °C reduces the ability of the roots to take-up the treatment preparation and therewith increases the chances of the root surviving the treatment, thereby resulting in the patient keeping their hair. According to WO 98/16176, the cooling treatment will preferably be continued for 1 to 5 hours.

One known head covering, in the form of a bonnet consisting of a soft-frozen gel block is placed on the patient. The block is heavy and often has a temperature of below -18 °C when the bonnet is placed on the head of the patient. Because the bonnet is inflexible and initially very cold, the device is uncomfortable. Another problem is that the frozen blocks quickly melt and do not therefore keep the temperature beneath the upper temperature limit during the whole treatment period. It is necessary for the patient to change bonnets, often as soon as about 45 minutes after having donned the head covering, when the temperature has risen from -18 °C to above +10 °C.

US 4,566,455 (Kramer), WO 98/16176 (Olofsson) and GB 2 323 915 (filed by the Applicant) all disclose a solution where the patient is fitted with a head covering that is continuously cooled. This head covering comprises two layers which define therebetween a space through which cooling fluid flows. The space in the head covering is connected by means of at least one hose to a cooling unit that supplies the head covering with fluid. The scalp is cooled if cold fluid is continuously applied at a relatively constant temperature during the whole of the treatment period. This obviates the need for the patient to place a stiff, supercooled covering on their head. The fluid inlet and outlet are preferably distributed over the head covering, so as to obtain good circulation and uniform cooling of the entire scalp.

The Kramer solution (US 4,566,455) regulates the temperature of the scalp cover using a thermistor attached to the top of the scalp of the patient, and a control unit arranged to receive the temperature from the thermistor and to activate a pump. The pump is activated to circulate liquid coolant when the temperature from the thermistor exceeds 20.5 °C, and is deactivated when the temperature falls below 20.5 °C.

The Olofsson solution (WO 98/16176) requires temperature regulation for each flow passage in the head covering to achieve a predetermined scalp temperature. This is achieved by providing a temperature sensor in each flow passage, and by providing a regulating device for individually regulating the temperature and/or flow of the fluid flowing through respective flow passages to achieve a desired set point value of +5 °C for example.

The Applicant's prior solution (GB 2 323 915, supplied under the name "*Model PSC-1*") comprises a powerful refrigerated cooling system which rapidly reduces the temperature of a liquid coolant in a bath to a pre-set temperature close to -10 °C. When the pre-set temperature has been reached, electronic sensors monitor and control the coolant to be around -10 °C. The coolant is pumped, at low pressure, through a scalp cooling cap. The scalp temperature is then reduced, to thereby prevent or alleviate hair loss. The success of the Applicant's scalp cooler relies on its ability to reduce the scalp temperature to a well documented and established level, of approximately 18 °C to 20 °C. However, the actual scalp temperature is difficult to measure through non-invasive methods with current technology.

The solution disclosed by Kramer does not measure scalp temperature accurately because of the effect of the hair and the inner surface of the scalp covering which will be much colder than the scalp. Also, fitting the thermistor is awkward, time-consuming and unreliable. The solution disclosed by Olofsson merely measures coolant temperature within the scalp cover. The Applicant's scalp cooler circulates coolant at a temperature (-10 °C as mentioned above) that has been found to give a high rate of success and which was developed on a basis of trial and error. The applicant's current solution is a one-size-fits-all approach which uses an extreme temperature setting to obtain the best chance of success.

However, not all patients feel comfortable undergoing treatment due to the coldness, and some stop treatment

2

altogether. In other cases, even when the treatment has been applied, excessive hair loss sometimes results.

In a related use, hands and feet may also be cooled to prevent damage during chemotherapy treatment, as explained in WO 2006/126059 (Fiomed APS), which proposes the use of gloves and socks which receive a circulated cooling fluid. Again, a temperature sensor is mounted on or within the glove or sock.

Other parts of the body, including the oral cavity, may also benefit from cooling. US Patent 5,509,801 (Nicholson) discloses an oral therapeutic apparatus comprising an oral device formed to be instertable within a patient's mouth and which receives a circulated cooling medium.

Further examples of the related art are described in US 2004/210283A1 (Rose Joseph Lorney et al), WO 99/44552 A1 (MTRE Advanced Technology et al), and US 2008/077202 A1 (Levinson Mitchell)

## SUMMARY OF THE INVENTION

An aim of the invention is to provide an improved body part temperature regulating apparatus. One aim of the invention is to improve tolerance rates among patients, and to improve success rates. In particular, the invention aims to provide an improved scalp cooler.

### Heat Transfer Statements of Invention

Broadly speaking, one aspect of the invention provides a new body part temperature regulating apparatus. The apparatus is arranged to regulate the temperature of a body part based on calculating the amount of heat transferred to or from the body part via a body part attachment. For example, the mass of a heat transfer fluid passed over the body part is determined, as is the difference in temperature of the heat transfer fluid across the body part attachment. The specific heat capacity of the heat transfer fluid is known in advance. Based on this information, a calculation is performed to establish the heat energy transferred to or from the body part to the heat transfer fluid during at least one predetermined time interval. The heat transfer process is then controlled based on the calculated heat energy extracted, preferably to achieve a predetermined heat transfer profile over a predetermined time period.

### Controller

More specifically, according to one aspect of the invention, there is provided a body part temperature regulating apparatus for regulating the temperature of a part of the human or animal body, the body part temperature regulating apparatus comprising:

a controller arranged to receive:

a first input from a first temperature sensor arranged to detect the temperature of a heat transfer fluid at a first location of the heat transfer fluid;

a second input from a second temperature sensor arranged to detect the temperature of the heat transfer fluid at a second location of the heat transfer fluid different to the first location;

a third input from a flow sensor arranged to detect the flow rate of the heat transfer fluid;

wherein the controller is arranged to determine the amount of heat transferred to the heat transfer fluid using the first, second and third inputs during a first predetermined period of time and to output a control signal to regulate the amount of heat transferred to the heat transfer fluid based on the determined amount of heat transferred.

In this way, an improved body part temperature regulating apparatus is provided which is able to control more accurately the temperature of the body part in question by measuring the heat energy extracted from the body part. Experiments show that the temperature of the body part is correlated to the amount of heat energy extracted over time.

The one-size-fits-all model can be replaced, and unnecessarily cold scalp temperatures or too high scalp temperatures can be avoided. This improvement will result in fewer patient drop-outs and fewer failures, leading to greater success rates.

The apparatus is also more accurate than those apparatuses of the prior art which measure directly the temperature of the heat transfer fluid in the body part covering or the body part covering surface itself.

As a useful side effect, the apparatus is more energy efficient, extracting closer to the minimum necessary heat from the body part. Also, if the temperature of the heat transfer fluid can be raised, such as in circumstances in which a user does not have a full head of hair, and hence has less insulation between the scalp, and the scalp covering, for example, the apparatus is able to achieve a pre-set condition much faster, thereby reducing waiting time. The controller is arranged to output the control signal to regulate the amount of heat transferred to the heat transfer fluid so that the amount of heat transferred follows a predetermined heat transfer characteristic.

In this way, flexibility can be introduced. For example, patients undergoing treatment on different drugs will be able to have different pre-programmed and desired heat transfer characteristics to suit each drug, respectively.

Preferably, the controller is arranged to determine the amount of heat transferred to the heat transfer fluid by:

(a) measuring the difference in temperature of the heat transfer fluid detected by the first temperature sensor and the second temperature sensor over a first predetermined period of time,
(b) determining the mass of the heat transfer fluid which has passed the temperature sensors over the first predetermined period of time from the flow rate detected by the flow sensor and a predetermined value indicating the mass per unit volume of the heat transfer fluid; and
(c) using a predetermined value corresponding to the specific heat capacity of the heat transfer fluid.

Preferably, the controller is arranged to determine the amount of heat transferred to the heat transfer fluid using the equation:

$$E = c \cdot m \cdot \Delta T$$

where:

$E$ = the heat energy transferred in joules (J);
$c$ = the known specific heat capacity of the heat transfer fluid in joules per kilogram kelvin (J/kg·K);
$m$ = mass of the heat transfer fluid in kilograms derived from the third input over the first predetermined period of time, and the density ($\rho$) of the heat transfer fluid;
$\Delta T$ = the difference in temperature in degrees kelvin (K) between the first input and the second input.

Preferably, the control signal is a temperature set-point for a heat exchanger. Preferably, the control signal is a flow control signal for a pump. Preferably, there are two control signals, the first control signal is a temperature set-point for a heat exchanger and the second control signal is a flow control signal for a pump. The first temperature sensor and the second temperature sensor are arranged to measure the temperature difference across a body part attachment when attached to the body part temperature regulating apparatus in use.

The apparatus comprises a heat exchanger, a pump, an outlet connection and an inlet connection all of which are connected together via interconnecting pipe work; a body part attachment for attachment to the human or animal body, and hosing which connects the body part attachment to the outlet connection and the inlet connection to achieve circulation of the heat transfer fluid, and the controller is arranged to control the heat exchanger, the pump, or both the heat exchanger and the pump. The first temperature sensor and the second temperature sensor are located so as to measure the temperature difference across the body part attachment.

Preferably, the first temperature sensor is disposed between the outlet of the heat exchanger and the inlet of the body part attachment, and the second temperature sensor is disposed between the outlet of the body part attachment and the inlet of the heat exchanger.

Preferably, the first temperature sensor is disposed between the outlet of the heat exchanger and the outlet connection, and the second temperature sensor is disposed between the inlet connection and the inlet of the heat exchanger.

Preferably, the flow sensor is provided between the heat exchanger and the outlet connection, or between the heat exchanger and the inlet connection.

Preferably, the heat exchanger is a refrigeration unit. Preferably, the refrigeration unit comprises a bath containing the heat transfer fluid, and refrigeration means to cool the heat transfer fluid.

The predetermined heat transfer characteristic is variable over time.

Preferably, the body part attachment is a scalp cooling attachment. Preferably, the body part temperature regulating apparatus is a scalp cooler.

Preferably, the controller is arranged to determine the body part temperature from the heat energy transferred measurement. Preferably, the body part temperature is a scalp surface temperature. Preferably, the scalp surface temperature is derived from the following equation:

$$SST = -0.079 \times E + 23.333$$

where;

SST = scalp surface temperature in degrees Celsius (°C);
-0.079 and 23.333 are constant values; and
E= the heat energy t transferred in joules (J) as defined above.

**System**

According to one aspect of the invention, there is provided a body part cooling apparatus for regulating the temperature of a part of the human or animal body, the body part cooling apparatus comprising:

a refrigeration and control unit; and

a body part cooling attachment;

wherein:

the refrigeration and control unit comprises: a heat exchanger, a pump, an outlet connection and an inlet connection all of which are connected together via interconnecting pipe work; an outlet temperature sensor, an inlet temperature sensor and a coolant flow rate sensor; and a controller arranged to receive respective signals from the inlet temperature sensor, the outlet temperature sensor and the coolant flow rate sensor and to control operation of the heat exchanger and the pump;

the body part cooling attachment comprises an attachment part for attaching to the human or animal body, and hosing which connects the attachment part to the outlet connection and the inlet connection of the refrigeration and control unit;

a coolant is disposed in the heat exchanger, pump, interconnecting pipe work, hosing and attachment part for circulation by the pump; and

the controller is arranged to monitor the difference in temperature of the coolant across at least the attachment part of the body part cooling attachment and to monitor the flow rate of the coolant through the attachment part, and to calculate the heat energy removed by the attachment part over a predetermined period of time, and to control the heat exchanger or the pump or both the heat exchanger and the pump to control the heat energy removed from the part of the human or animal body by the attachment part based on the calculated heat energy extracted.

Other preferred features are omitted here for brevity, but are equally applicable from the other aspects of the invention and description where appropriate.

**Method**

There is also described a method for controlling a body part temperature regulating apparatus for regulating the temperature of a part of the human or animal body, the method comprising:

receiving:

a first input from a first temperature sensor arranged to detect the temperature of a heat transfer fluid at a first location of the heat transfer fluid;

a second input from a second temperature sensor arranged to detect the temperature of the heat transfer fluid at a second location of the heat transfer fluid different to the first location;

a third input from a flow sensor arranged to detect the flow rate of the heat transfer fluid; and

determining the amount of heat transferred to the heat transfer fluid using the first, second and third inputs during a first predetermined period of time; and

regulating the amount of heat transferring to the heat transfer fluid based on the determined amount of heat transferred.

Preferably, the regulating the amount of heat transferring causes the amount of heat transferred to follow a predetermined heat transfer characteristic.

Preferably, the determining comprises:

(a) measuring the difference in temperature of the heat transfer fluid detected by the first temperature sensor and the second temperature sensor over a first predetermined period of time,

(b) determining the mass of the heat transfer fluid which has passed the temperature sensors over the first predetermined period of time from the flow rate detected by the flow sensor and a predetermined value indicating the mass per unit volume of the heat transfer fluid; and

(c) using a predetermined value corresponding to the specific heat capacity of the heat transfer fluid.

Preferably, the determining uses the equation:

$$E = c \cdot m \cdot \Delta T$$

where:

$E$ = the heat energy transferred in joules (J);
$c$ = the known specific heat capacity of the heat transfer fluid in joules per kilogram kelvin (J/kg·K);
$m$ = mass of the heat transfer fluid in kilograms derived from the third input over the first predetermined period of time, and the density ($\rho$) of the heat transfer fluid;
$\Delta T$ = the difference in temperature in degrees kelvin (K) between the first input and the second input.

Preferably, the regulating comprises outputting a temperature set-point for a heat exchanger. Preferably, the regulating comprises outputting a flow control signal for a pump. Preferably, the regulating comprises outputting a temperature set-point for a heat exchanger and a flow control signal for a pump. The first temperature sensor and the second temperature sensor are located so as to measure the temperature difference across the body part attachment. The predetermined heat transfer characteristic is variable over time.

Preferably, the body part attachment is a scalp cooling attachment. Preferably, the body part temperature regulating apparatus is a scalp cooler.

Preferably, the method comprises calculating the body part temperature from the heat energy transferred determination. Preferably, the body part temperature is a scalp surface temperature. Preferably, the scalp surface temperature is derived from the following equation:

$$SST = -0.079 \times E + 23.333$$

where;

SST = scalp surface temperature in degrees Celsius (°C);
-0.079 and 23.333 are constant values; and
$E$ = the heat energy t transferred in joules (J) as defined above.

The amount of heat transferred is regulated by a predetermined heat transfer characteristic which varies over time.

In particular it should be noted that the predetermined heat transfer characteristic varies depending on the drug which is administered to the patient undergoing treatment and whose body part is being cooled.

The predetermined heat transfer characteristics will be selected according to the drug or combination of drugs with which the patient is being treated. The predetermined heat transfer characteristic may also depend on the dosage of the or each drug which is administered.

In preferred embodiments the heat transfer characteristics are predetermined by reference to experimental data. For example it may be known that a particular drug or drug combination provides a high blood concentration of the drug or drugs between 30 and 60 minutes after completion of administration. Thus cooling would suitably be increased during this period.

The extent of cooling at any particular time may be increased by lowering the temperature of the heat transfer fluid and/or by increasing the flow rate of the heat transfer fluid. The predetermined heat transfer characteristics may therefore depend on the pharmacokinetic profile of the drug or drug combination which is being administered.

The pharmacokinetic profile or a drug or drub combination can therefore be used to determine suitable times and temperatures of cooling. This allows more intense periods of cooling to be carried out for shorter periods thus increasing tolerance of the treatment.

The predetermined heat transfer characteristics may suitably be determined by reference to experimental data. This experimental data may be empirical observations from treatment of previous patients with the same or similar drug or drug combinations. The experimental data may alternatively and/or additionally involve the use of biological assays. For example *in vitro* cell culture models may be used to determine the effects of chemotherapy drugs on cancer cells and on fast-dividing epithelial cells in the root of the hair follicles of the human skin. The way in which the effect of the drug varies with temperature, dosage and time after administration can be examined and this information can then be used to design an appropriate cooling regime for treatment with a particular drug or drug combination.

Thus the method of the present invention may first involve a step prior to treatment of determining appropriate heat transfer characteristics. This step suitably involves consideration of experimental data.

There have been some concerns regarding possible risks of scalp metastasis when scalp cooling is used during administration of chemotherapy drugs in the treatment of cancer.

Although such a link has not been definitively proven, the shorter cooling times used in the method of the present invention may help overcome any fears people have.

**Cap**

According to another aspect of the invention, there is provided a body part attachment for use with a body part temperature regulating apparatus, the body part attachment comprising:

a length of tubing, wherein the outside diameter of the tubing is expandable to increase the surface area of the body part attachment when in contact with the body part in use.

In this way, the body part attachment is capable of expanding under normal usage conditions. In other words, the body part attachment is self-expanding. Better contact can be achieved between the body part attachment and the body part, thus improving the rate of heat transfer between the body part attachment and the body part. This in turn allows a more flexible apparatus able to achieve a wider range of heat transfer rates.

Preferably, the body part attachment is arranged so that expansion of the tubing increases the outside surface of the length of tubing in contact with the body part in use by at least twofold. Preferably, the body part attachment is arranged so that expansion of the tubing increases the outside surface of the length of tubing in contact with the body part in use by at least threefold.

Preferably, the outside diameter is expandable by between 5% and 50% under a normal operating pressure. More preferably, the outside diameter is expandable by between 10% and 30% under the normal operating pressure. Preferably still, the outside diameter is expandable by between 15% and 25% under the normal operating pressure. Most preferably, the outside diameter is expandable by substantially 20% under the normal operating pressure. Preferably, the normal operating pressure is between 100 kPa and 150 kPa. More preferably, the normal operating pressure is between 110 kPa and 130 kPa. Most preferably, the normal operating pressure is between 115 and 120 kPa.

Preferably, the tubing has a wall thickness of between 0.5 mm and 3 mm. Most preferably, the tubing has a wall thickness of substantially 1 mm. Preferably, the tubing has an external diameter of between 6 mm and 10 mm. More preferably, the tubing has an external diameter of between 7 mm and 9 mm. Most preferably, the tubing has an external diameter of substantially 8 mm. Preferably, the tubing has an internal diameter of between 4 mm and 8 mm. More preferably, the tubing has in internal diameter of between 5 mm and 7 mm. Most preferably, the tubing has an internal diameter of 6 mm.

In an example embodiment, the length of tubing is wound to conform to a shape of the body part, and has an inlet and an outlet. Preferably, the length of tubing is a continuous single length.

Preferably the inlet and outlet are adjacent each other. Preferably, the tubing is arranged to have a minimum curve radii of above 3 mm and preferably 5 mm.

Preferably, the tubing is made from silicone rubber. Preferably, the tubing is a general purpose silicone rubber.

Preferably, the tubing is made from a silicone rubber having a hardness rating of Shore A 40.

Preferably, the body part attachment has a resilient cover which covers substantially the tubing which is arranged to abut the body part in use. Preferably, the cover is made from a treated cotton or a plastics material.

Preferably, the body part attachment is a cap for covering a scalp. Preferably, the body part temperature regulating apparatus is a scalp cooler.

According to another aspect of the invention, there is provided a method of using a body part attachment of a body part temperature regulating apparatus, the method comprising:

> pumping a heat transfer fluid through the body part attachment at a pressure which increases the surface area of the body part attachment in contact with the body part.

Preferably, the pumping causes the surface area of the body part attachment in contact with the body part to increase by at least twofold. More preferably, the pumping causes the surface area of the body part attachment in contact with the body part to increase by at least threefold.

Preferably, the body part attachment comprises tubing, and the surface area of the body part attachment to increase by expanding the outside diameter of the tubing by one of between 5% and 50%, 15% and 30%, and substantially 20%.

Preferably, the pumping creates an internal pressure of one of between 100 kPa and 150 kPa, 110 kPa and 130 kPa and 115 kPa and 120 kPa in the body part attachment to cause the surface area to increase.

Preferably, the body part attachment is as described above. Preferably, the body part attachment is a cap for covering a scalp. Preferably, the body part temperature regulating apparatus is a scalp cooler.

Preferably, the body part attachment and method are used with the body part temperature regulating apparatus, body part cooling apparatus and associated method described above.

## Coolant

Preferably the heat transfer fluid is a coolant. Preferably the coolant is a liquid. Any suitable liquid may be used. A liquid will be typically selected due to its low freezing point and minimal effect of low temperatures on viscosity. Preferably the primary coolant has a freezing point of less than 0°C, preferably less than -5°C, more preferably less than -10°C, suitably less than -12°C, preferably less than -13°C.

Preferably the coolant is an aqueous based liquid. It is known that mixtures of glycol compounds and water have low freezing points and can be used as coolants. Such mixtures could be used as coolants in the present invention. However these compositions have relatively high viscosity and can be difficult to pump. In addition compositions of high viscosity provide less efficient heat exchange. At present, the applicant achieves a success rate of approximately 80% using known glycol mixtures. The applicant has developed an improved coolant composition which can be used as the coolant in the present invention.

Preferably the coolant is an aqueous composition comprising a freeze point depressant, a corrosion inhibitor and water.

The freeze point depressant is preferably the salt of an aliphatic or aromatic carboxylic acid having up to 20 carbon atoms. Preferably it is a salt of an aliphatic carboxylic acid, preferably having up to 15, more preferably up to 12 or up to 10 carbon atoms.

Suitably the freeze point depressant comprises the salt of an aliphatic organic acid having 1 to 8 carbon atoms and 1 to 3 carboxylic acid residues. When more than one carboxylic acid residue is present, the salt may be a monosalt, or a disalt or a trisalt. Where more than one cationic counterion is present, these may be the same or different or in some cases may comprise a polyvalent cation.

The organic acid may be substituted, for example it may include hydroxyl substituents. Preferably it is unsubstituted. Preferably the freeze point depressant includes only a single acid moiety. Most preferably the freeze point depressant comprises an organic acid having 1 carboxylic acid group and 1 to 4 carbon atoms. Preferably the freeze point depressant is a sodium or potassium salt of an organic acid having 1 or 2 carbon atoms. Most preferably it is potassium formate.

The corrosion inhibitor may also act as a freeze point depressant and is present in addition to the other freeze point depressant material.

The freeze point depressant and corrosion inhibitor may be regarded as acting synergistically to provide a desired depression of the freeze point.

It is a feature of the present invention that the temperature at which the coolant composition freezes may be varied by varying the amount of freeze point depression present in the composition. Preferably the freeze point depressant is present in the composition in an amount of from 5 to 30 gdm$^{-3}$ for each 1°C depression of the freezing point. The freezing point depression is measured with respect to pure water. Thus if it is desired to depress the freezing point by 1°C, from 5 to 30 gdm$^{-3}$ of freeze point depressant is added; for a depression of 2°C, from 10 to 60 gdm$^{-3}$ of freeze point depressant is added, and so on.

Suitably the freeze point depressant is present in an amount of from 10 to 25 gdm$^{-3}$ for each °C depression of the freezing point.

The composition is an aqueous composition. Thus depression of the freezing point of the composition refers to depression of the freezing point of water. Hence a depression of 3°C will lead to a composition which freezes at -3°C.

In preferred embodiments, the freeze point depressant is present in an amount to provide a freezing point of from -12 to -20°C, for example about -15°C.

If the coolant were to be used to cool a body part attachment to, for example, a minimum of -10°C it would be desirable to use a composition which freezes at -15°C to prevent accidental over cooling if the apparatus malfunctions. This is because in the apparatus the refrigeration evaporator coils are very cold, approx -25°C. This means that the coolant, when formulated to freeze at -15°C, actually freezes at the evaporator coils when the body part attachment is at -10°C. The coolant will be at -4°C in the majority of cases, but may be lower with patients with thick hair for instance, but the coolant will provide a limit of -10°C at the patient. This provides an inbuilt safeguard in the event of a fault situation where the apparatus continues to cool the coolant.

The coolant composition may include any suitable corrosion inhibitor. Examples of compounds suitable for use as corrosion inhibitors include hexamine, phenylenediamine, dimethylethanolamine, cinnamaldehyde, condensation products of aldehydes and amines (imines), chromates, nitrites, phosphates, phosphonates, sodium benzoate, sodium triazoles and organic acids.

Preferred corrosion inhibitors for use in the present invention are phosphate compounds, in particular ammonium, substituted ammonium or alkali metal salts of phosphoric acid. One or more cationic counterions may be present. Preferably the corrosion inhibitor comprises a salt of phosphoric acid with sodium and/or potassium. Most preferably it comprises dipotassium phosphate.

In some embodiments may be used a mixture of corrosion inhibitors. Preferably however dipotassium phosphate is the major corrosion inhibitor present. Other corrosion inhibitors, where present, are present in minor amounts compared with dipotassium phosphate, for example less than 10% by weight. Suitably dipotassium phosphate is the only corrosion inhibitor present. When two or more corrosion inhibitors are present, these compounds preferably act synergistically. In some a heterocyclic corrosion inhibitor may also be present. Preferred are nitrogen-containing heterocyclic compounds.

An aromatic heterocyclic corrosion inhibitor may be used. Most suitable are azole compounds, especially triazoles. Examples include benzatriazoles, and substituted benzatriazoles, especially tolutriazole. However, in preferred embodiments, the composition does not include an aromatic heterocyclic corrosion inhibitor.

The corrosion inhibitor may comprise an organic acid. Preferred organic acids include those having 1 to 10, preferably 1 to 6 carbon atoms, and 1 to 3 carboxylic acid residues. Most preferred are organic acids having 1 to 3 carbon atoms and one carboxylic acid group. The corrosion inhibitor may comprise an organic acid selected from formic acid, acetic acid and a mixture thereof.

The corrosion inhibitor is preferably present in the primary coolant composition in an amount of at least 0.1 gdm$^{-3}$, preferably at least 0.5 gdm$^{-3}$, preferably at least 1 gdm$^{-3}$, more preferably at least 2 gdm$^{-3}$, and most preferably at least 3 gdm$^{-3}$. The corrosion inhibitor is preferably present in an amount of up to 8 gdm$^{-3}$, preferably up to 7 gdm$^{-3}$, more preferably up to 6 gdm$^{-3}$, suitably up to 5 gdm$^{-3}$.

The above concentrations refer to the total amount of all of corrosion inhibitors present in the primary coolant composition.

Suitably the ratio of freeze point depressant to corrosion inhibitor may be varied to control the temperature at which the composition freezes. This is usually achieved by varying the amount of freeze point depressant.

Preferably the freeze point depressant and corrosion inhibitor together comprise at least 50 wt% of all solids dissolved in the coolant composition of the present invention, more preferably at least 70 wt%, preferably at 90 wt%, preferably at least 95 wt%, for example at least 97 wt%.

Optionally the cooling composition of the present invention may comprise further components.

In some embodiments, the composition of the present invention may further comprise a deposit combatant. The deposit combatant may comprise a single chemical moiety or it may include a mixture of compounds. The term deposit combatant hereinafter refers to the total amount of all such compounds.

The term deposit combatant includes antiscalant compounds. This term includes dispersant compounds. Suitable combatants may act as an antiscalant and a dispersant. These are preferably compound(s) which are able to sequester certain cations, for example magnesium and calcium to maintain them in solution and prevent deposits building up on the internal surfaces of the apparatus. Any suitable sequestering agent may be used as a deposit combatant and such compounds will be well known to the person skilled in the art.

Preferred deposit combatants include homopolymers and copolymers of polycarboxylic acids and their partially or completely neutralized salts, monomeric polycarboxylic acids and hydroxycarboxylic acids and their salts, phosphates and phosphonates, and mixtures of such substances. Preferred salts of the abovementioned compounds are the ammonium and/or alkali metal salts, i.e. the lithium, sodium, and potassium salts, and particularly preferred salts is the sodium salts.

Suitable polycarboxylic acids are acyclic, alicyclic, heterocyclic and aromatic carboxylic acids.

The deposit combatant most preferably comprises a polymer of acrylic acid or a salt thereof. Preferably it comprises a polyacrylate compound. Preferably the deposit combatant comprises a sodium polyacrylate. Suitable polyacrylates are those having a molecular weight of from 200 to 25000, preferably from 500 to 10000, for example 800 to 5000 or 1000 to 3000. Preferred deposit combatants include those sold by Cytec, for example under the trade mark Cytec P70. Suitably such compounds have been approved for use in drinking water.

The composition may include a component having antimicrobial properties. This may be provided by the freeze point depressant. For example potassium formate has antimicrobial properties.

Alternatively, the composition may include an additional antimicrobial component. For example it may include a biocide.

Any suitable biocide may be used and will be known to the person skilled in the art.

Thus the coolant used in the present invention is preferably not a glycol-water mixture. However, the coolant may comprise less than 20 wt% glycol, preferably less than 10 wt%, more preferably less than 5 wt% and most preferably less than 2 wt%, for example less than 1 wt%. In particular the coolant may comprise less than 20% glycol, or more preferably less than 15% glycol, or more preferably less than or approximately 13% glycol which would give a -4°C freezepoint.

One preferred coolant composition of the present invention is available under the trademark OrbisC.

The present invention provides the use of an aqueous composition comprising a freeze point depressant, a corrosion inhibitor and water to regulate the temperature of a body part. Preferably the body part is a scalp. Preferred features of the aqueous composition are as previously described herein. In especially preferred embodiments the composition is used in the apparatus of the present invention.

The present invention may further provide a heat transfer fluid as described herein.

## BRIEF DESCRIPTION OF THE DRAWINGS

For a better understanding of the invention, and to show how example embodiments may be carried into effect, reference will now be made to the accompanying drawings in which:

Fig. 1 is a perspective view of a body part temperature regulating apparatus according to the invention;

Fig. 2 is a schematic overview of the body part temperature regulating apparatus of Fig. 1 having a single cap attached thereto; and

Fig. 3 is a schematic overview of the body part temperature regulating apparatus of Fig. 1 having two caps attached thereto in parallel;

Fig. 4 is a schematic overview of the boy part temperature regulating apparatus of Fig. 2 having two caps attached thereto in series;

Fig. 5 is a flow chart of a method of preparing the body part temperature regulating apparatus of Fig. 1;

Fig. 6 is a flowchart of a method of regulating the amount of heat transferred;

Fig. 7 is a flowchart showing the method of Fig. 6 in more detail;

Fig. 8 is a graph of a flow rate comparison of a first coolant (glycol) and a second coolant (OrbisC™);

Fig. 9 is a graph illustrating an example relationship between heat energy extracted and bath set-point temperature for a test patient;

Fig. 10 is a graph illustrating the relationship between scalp surface temperature and bath set-point for a test patient;

Fig. 11 is a more detailed side view of a body part attachment shown in Fig. 1;

Fig. 12 is a partial cross-sectional view of the body part attachment shown in Fig. 10 when adjacent a patient's head and no heat transfer fluid is flowing; and

Fig. 13 is a partial cross-sectional view of the body part attachment shown in Fig. 10 when adjacent a patient's head and when heat transfer fluid is flowing.

**DETAILED DESCRIPTION OF THE EXAMPLE EMBODIMENTS**

By way of example to illustrate the invention, a non-limiting embodiment is now described.

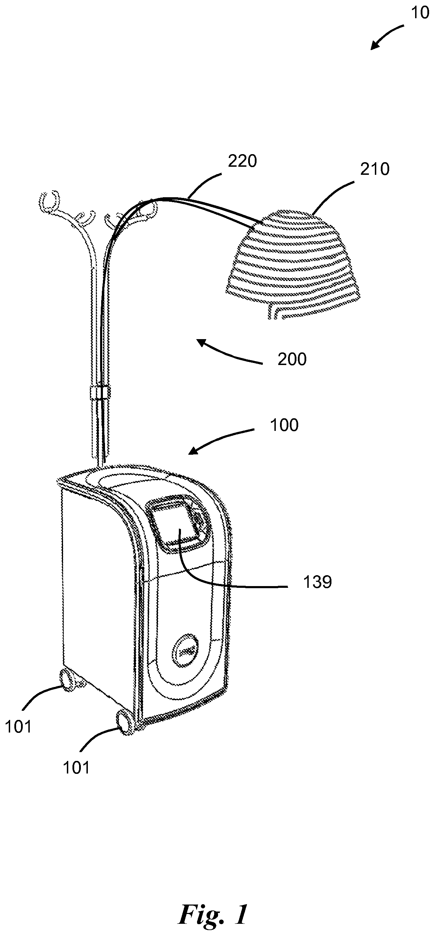

Fig. 1 is a perspective view of a body part temperature regulating apparatus 10 according to the invention. In this example embodiment, the body part temperature regulating apparatus 10 is a scalp cooling apparatus 10. The scalp cooling apparatus 10 comprises a refrigeration and control unit 100 together with a body part attachment, specifically a scalp cooling attachment 200.

The refrigeration and control unit 100 is a portable unit mounted on casters 101 so that the portable unit can be pushed easily from room to room within, say, a hospital. The refrigeration and control unit 100 comprises a mains power connection and a power on/off switch a neither of which are shown in Fig. 1. A user interface 139 is also provided.

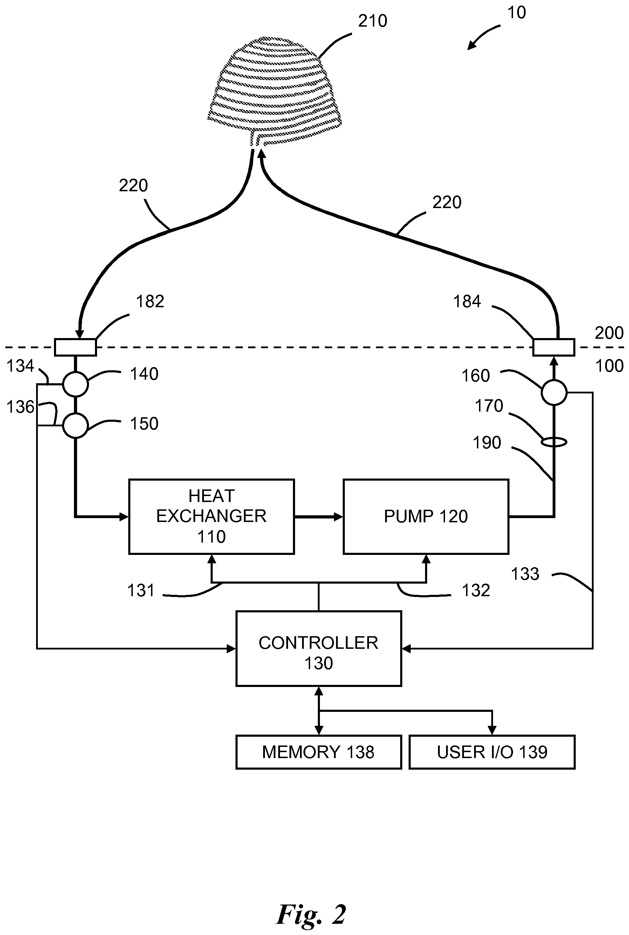

Fig. 2 shows a schematic overview of the body part temperature regulating apparatus 10.

The refrigeration and control unit 100 comprises a heat exchanger 110, a pump 120 and a controller 130 arranged to output control signals via outputs 131 and 132 to control operation of the heat exchanger 110 and pump 120, respectively.

The refrigeration and control unit 100 further comprises two external connections, namely an outlet connection 184 and a return connection 182. Interconnecting pipe work 170 is arranged to connect the return connection 182 to the heat exchanger 110, and to connect the heat exchanger 110 to the pump 120, and to connect the pump 120 to the outlet connection 184. The interconnecting pipe work 170, heat exchanger 110 and pump 120 are filled with a heat transfer fluid, in this case a coolant 190. The direction of the arrow heads of the interconnecting pipe work 170 shows the direction of flow of the coolant 190.

Additionally, a first temperature sensor 160, or an outlet temperature sensor, is arranged in the interconnecting pipe work 170 between the pump 120 and the outlet connection 184. A second temperature sensor 140, or a return temperature sensor, is located in the interconnecting pipe work 170 between the return connection 182 and the heat exchanger 110. A flow rate sensor 150 is located in the interconnecting pipe work 170 between the return connection 182 and the outlet connection 184. In this example, the flow rate sensor 150 is located in the interconnecting pipe work 170 between the return connection 182 and the heat exchanger 110.

The exact position of the first temperature sensor 160, the second temperature sensor 140 and the flow rate sensor 150 are not critical to the invention. The first temperature sensor 160 and the second temperature sensor 140 should however be located to measure the temperature difference across the body part attachment 200 in use and at the very least be at opposite sides of the heat exchanger 110. In this preferred example, the first temperature sensor 160 and the second temperature sensor 140 are positioned within the refrigeration and control unit 100 adjacent the respective external connections 182, 184 for ease of manufacture and use.

The outlet temperature sensor 160, the return temperature sensor 140 and the flow rate sensor 150 are arranged to provide signals corresponding to each respective measurement to the controller 130, which has corresponding inputs 133, 134 and 136 to receive the signals shown by input lines on Fig. 2.

The controller 130 is also connected to a computer memory 138 and the user interface 139.

In more detail, the heat exchanger 110 is a hermetically sealed refrigeration unit using a small fraction of a horsepower (around 1/5th of a horsepower) compressor, a standard condenser unit with a copper coiled evaporator, and uses a standard CFC free 134A refrigerant. The pump 120 is a standard semi-submersible column pump capable of up to 250 ml/s flow and up to 117.65 kPa (or 12 meters of head). Normally, the pump 120 is used at approximately 25 ml/s with 19.61 kPa (or 2 meters of head). The inlet temperature sensor 140 and the outlet temperature sensor 160 are negative temperature coefficient types with tolerances of +/- 0.5 °C at 0 °C. The flow meter 150 is a turbine-type flow meter giving 1420 pulses per litre with an accuracy of +- 0.75% at 25 ml/s.

In this example, the coolant 190 is a specialist coolant disclosed earlier in the statements of invention. The specialist coolant has been found to have surprising advantages when used in the body part temperature regulating apparatus 10 disclosed herein. Not least, the specialist coolant is less viscous, especially at temperatures below 0 °C, than glycol-based coolants. The specialist coolant provides increased flow, and greater heat transfer for a given bath temperature than glycol-based coolants, which advantageously results in higher bath temperatures to achieve the same amount of heat transfer.

In particular, the coolant 190 has a dynamic viscosity ($\mu$) of 7.35 mPa·s and a kinematic viscosity ($v$) of 6.2 mm$^2$/s, both at -10 °C, and a dynamic viscosity ($\mu$) of 2.752 mPa·s and a kinematic viscosity ($v$) of 2.384 mm$^2$/s, both at -4 °C, which leads to flow rates of 27 ml/s versus 9 ml/s for a 33% monopropylene glycol/water mixture.

The scalp cooling attachment 200 comprises a cap 210 for placing over the scalp of a patient. In practice, many different sizes of cap 210 are provided with the apparatus 10, so that the patient can find a cap 210 having a best fit.

The scalp cooling attachment 200 also comprises two lengths of flexible hosing 220 which connect the scalp 210 to the outlet connection 184 and the return connection 182, respectively. The flexible hosing 220 terminates at the cap 210 in such a way as to promote good fluid movement of the coolant 190 around the cap 210, and thereby promote efficient heat transfer from the patient's scalp.

The outlet connection 184 and the return connection 182 are provided with quick-release couplings for attachment to the flexible hosing 220.

The controller 130 is arranged to receive a first input from the first temperature sensor 160 via input 133, a second input from the second temperature sensor via input 134 and a third input from the flow sensor 150 via input 136. The controller 130 is arranged to determine the amount of heat transferred to the coolant 190 during a first predetermined period of time.

In this example, the controller 130 is arranged to determine the amount of heat energy transferred over a first predetermined period of time to the coolant 190 using the equation:

$$E = c \cdot m \cdot \Delta T \quad \text{(Equation 1)}$$

where:

$E$ = the heat energy transferred in joules (J)

$c$ = the known specific heat capacity of the coolant 190 in joules per kilogram kelvin (J/kg·K);

$m$ = mass of the coolant 190 in kilograms derived from the third input, that is the volumetric flow rate ($Q$) in cubic meters per second ($m^3$/s) over the first predetermined period of time, and the density ($\rho$) of the coolant 190 stored in the computer memory 138;

$\Delta T$ = the difference in temperature in degrees kelvin (K) between the first input, that is the outlet temperature T1 and the second input, that is the return temperature T2.

The controller 130 is arranged to compare the energy transferred over the first predetermined period of time with either a predetermined value or a predetermined heat transfer characteristic stored in the computer memory 138, and to output a control signal 131, 132 to cause the amount of heat transferred to approach the predetermined value or to follow the predetermined heat transfer characteristic. In one example, the controller 130 may output an alarm via the user interface 139 to prompt a user to step up or step down the amount of heat being transferred, again via the user interface 139. In another example, the controller 130 automatically adjusts the rate of heat transfer based on a predetermined algorithm. This may be to meet the predetermined value of heat transfer, or to follow the predetermined heat transfer characteristic. In other words, the controller 130 creates a control loop to regulate the energy transferred from the human or animal body part based on the determined heat transferred.

In this example, the control signal is a temperature set-point for the heat exchanger 110, so that the temperature of the coolant 190 leaving the heat exchanger 110 and entering the cap 210 is raised or lowered to increase or decrease the rate of heat energy transferred to the coolant 190, respectively.

Alternatively, the control signal may be a flow rate set-point or a pump speed control signal for controlling the pump 120, so that the mass of coolant 190 passing through the cap 210 per second may be raised or lowered to increase or decrease the rate of heat energy transferred to the coolant 190, respectively.

The controller 130 may output a combination of both a temperature set-point and a flowrate set-point or a pump speed control signal.

Alternatively, or additionally, the controller 130 may cause the user interface 139 to output a suggestion that the cap 210 is checked for correct fitting or advice that a conditioner is used on a patient's hair to improve heat transfer.

The predetermined heat transfer characteristic may be a fixed rate of heat transfer, or may be a variable rate of heat transfer. For example, the heat transfer characteristic may require a predetermined amount of energy to be transferred over the whole of a treatment programme, and for the rate of heat transfer not to change during the programme. Alternatively, a variable rate of heat transfer may be used during the programme. This could have the form of a positive or negative ramp, or of some other useful shape such as a bell curve or an inverse bell curve.

In this example, the controller 130 is arranged to determine the scalp temperature from the value of heat energy transferred. This relationship has been found empirically from several tests using the apparatus 10, which are explained later.

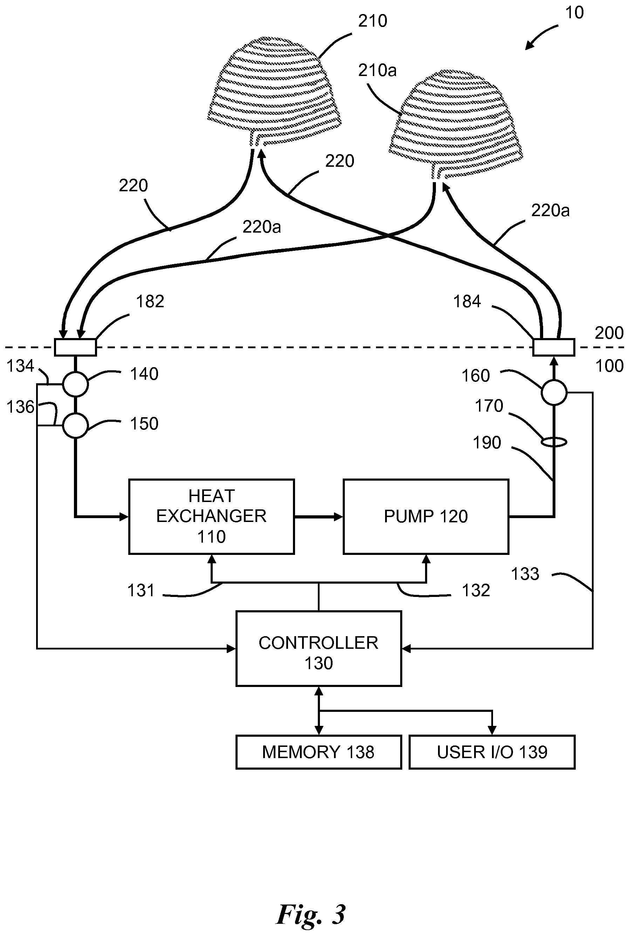

Fig. 3 is a schematic drawing of a variant of the body part temperature regulating apparatus 10 of Figs. 1 and 2. Here, a second cap 210a is shown connected in parallel to the cap 210 of Figs. 1 and 2 by second flexible hosing 220a. The outlet connection 184 and the inlet connection 182 have an additional port for this purpose (not shown) and are configured to connect in parallel both body part attachments 210 and 210a via respective flexible hosing 220 and 220a. When the second cap 210a is not connected, then coolant 190 flows through only the port in the external connection 184 and 182 which is connected to flexible hosing 220. No coolant 190 is lost.

As mentioned earlier, flow rates of approximately 28 ml/s are achieved with one cap 210 connected to the refrigeration and control unit 100. When two caps 210 and 210a are connected in parallel, as shown in Fig. 3, then flow rates of approximately 17 ml/s are achieved.

A small bypass circuit is not shown which limits the pressure into a cap 210, for example when the return connection is not made. The pressure is limited to 138 kPa (or 20 psi).

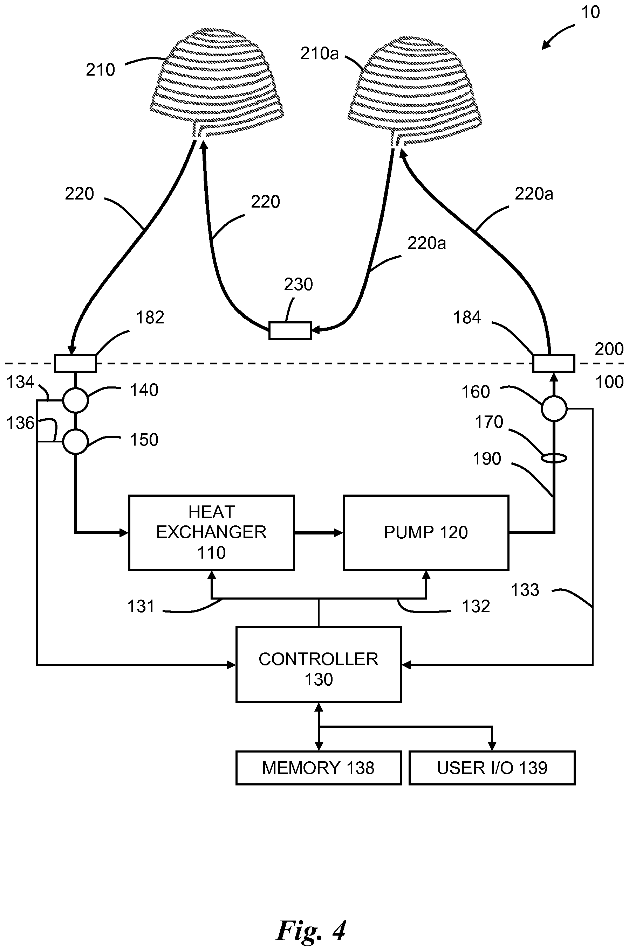

In another variant shown in Fig. 4, the two caps 210 and 210a can be connected in series by a connector 230, and flow rates of 14 ml/s are achieved.

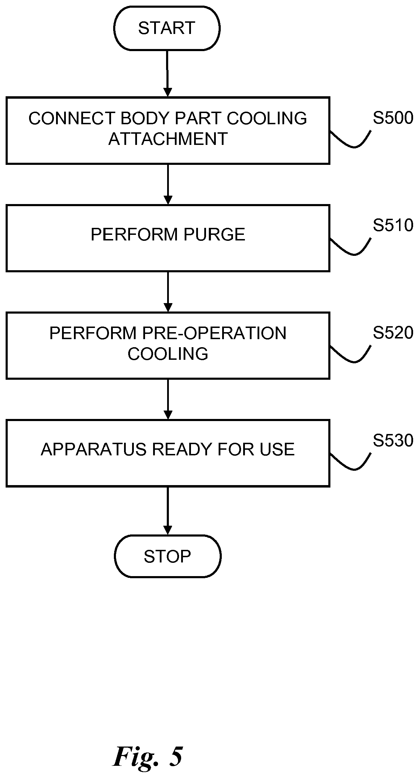

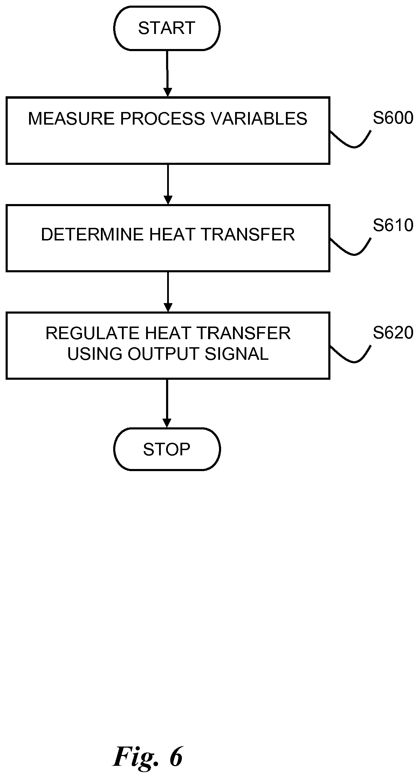

A method of operation of the scalp cooling apparatus 10 is now described, with reference to Fig. 5 and Fig. 6.

Fig. 5 describes pre-operation steps.

Prior to operation, the scalp cooling attachment 200 is connected (S300) to the refrigeration control unit 100. A pre-operation purging process (S310) is optionally carried out to remove any air bubbles in the coolant 190 and pump 120. Also, a pre-operation cooling process (S320) takes place to bring the coolant temperature, measured using the outlet temperature sensor 160, or the return temperature sensor 140, to a preset value or range. That is, this preset value may differ depending on the patient, the type of coolant 190, and the type of cooling program used. Types of cooling program will be briefly discussed later in this document.

Once the pre-operation steps are completed, the apparatus 10 is arranged to begin treatment of the patient.

Fig. 6 is a flow chart which describes the main measurement and control steps according to the invention.

Firstly, the process variables required to determine the heat energy transferred to the coolant 190 are measured (S600).

Then, the amount of heat transferred to the coolant 190 from the patient is determined (S610).

Finally, an output signal is used to regulate the heat transfer rate (S620)

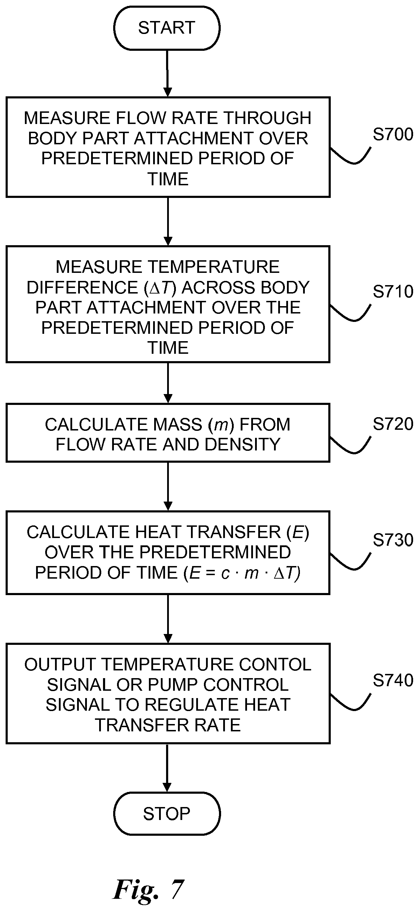

Fig. 7 is a flow chart which shows the main measurement and control steps according to Fig. 6 in more detail.

At step S700, the flow rate sensor 150 measures the flow rate of coolant 190 through the interconnecting pipe work 170, and thereby through the cap 210, over a predetermined period of time.

At step S710, the outlet temperature and inlet temperature are measured to determine the temperature difference across the cap 210, over the predetermined period of time. Steps S700 and S710 can be in reverse order.

At step S720, the controller 130 calculates the mass of coolant 190 flowing through the cap 210, using pre-determined and pre-stored information on the type of coolant 190 used in the apparatus 10. In this example, the density of the coolant 190 at the circulating temperature measured by one of the temperature sensors 140, 160 is used for this calculation. Either one of the outlet temperature or the return temperature is measured using the outlet temperature sensor 160 or the return temperature sensor 140, respectively. In practice, both temperatures are measured at the same, or at around the same time as the flow rate. The density of the coolant 190 is determined form a look-up table in the computer memory 138 corresponding to the coolant temperature.

At step S730, the heat transfer rate is calculated using the difference between the outlet temperature and the return temperature, the mass of coolant 190 passing through the cap 210, and the specific heat capacity of the coolant 190. The amount of heat transferred over the first predetermined period of time is calculated and stored. Also, the total heat transferred since the beginning of the treatment is also calculated and stored. Storage of this data is within the computer memory 138 connected to the controller 130.

At step S740, the controller 130 is arranged to control one or both of the heat exchanger 110 and the pump 120 in order to vary the heat transfer rate to achieve one or more target values over one or more pre-determined periods of time. For example, decreasing the temperature of the coolant 190 via the heat exchanger 110 will increase the rate of heat transfer from the scalp of a patient to the cap 210 when the coolant 190 is colder than the patient. Conversely, increasing the temperature of the coolant 190 via the heat exchanger 110 will decrease the heat transfer rate from a scalp of the patient to the cap 210. Likewise, increasing the flow rate via pump 120 will increase the heat transfer rate, and conversely, decreasing the flow rate via the pump 120 will decrease the heat transfer rate.

Pre-programmed algorithms can be set in the controller 130 to control this process. For example, one example program may require a constant heat transfer rate over the whole treatment period. To achieve this, the coolant temperatures may need to vary depending on factors such as the ability of the patient to replenish lost heat energy.

Additionally, a program can be used which gradually increases, or decreases, the rate of heat transfer from the beginning of a treatment to the end of a treatment. In one program which is envisaged, the heat transfer rate can be varied in any fashion throughout the treatment period.

In this way, a treatment suitable to a particular patient can be created, to optimise the chances of success of the treatment.

PRACTICAL TESTS AND RESULTS

In order to test the assumptions and theories underlying the invention, various practical tests were undertaken which are now explained.

The first test compared two coolants having different specific heat characteristics and viscosities.

The first coolant used in the test was a known coolant comprising a 33% monopropylene glycol/water mix (glycol) having a first specific heat capacity ($c_1$ = 3860 at -10 °C) and a first viscosity ($\mu$ = 14.9 mPa s at -10 °C).

The second coolant used in the test was a new coolant 190 discussed above (OrbisC™) with a specific heat capacity ($c_2$ = 3380 at -10 °C) and a second viscosity ($\mu$ = 7.35 mPa·s at -10 °C).

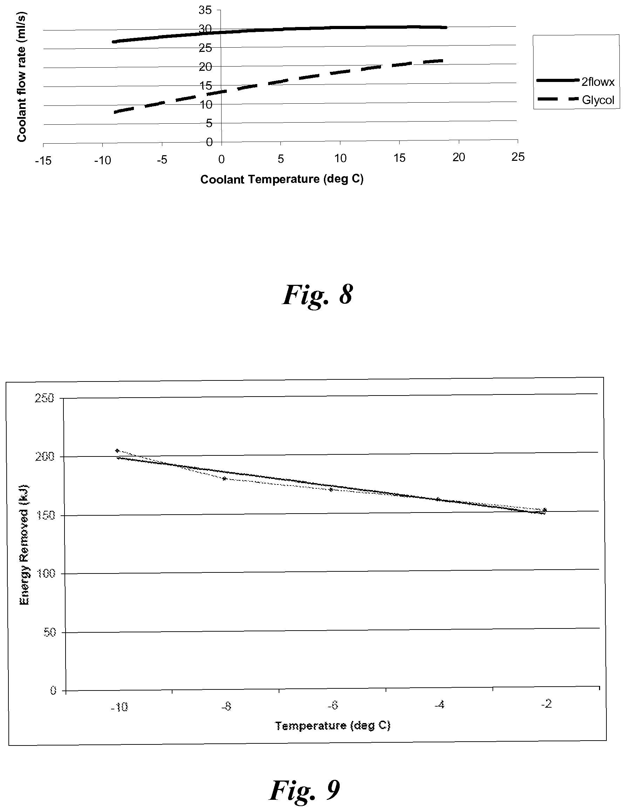

Fig. 8 shows a graph of a flow rate comparison of the first coolant (glycol) and the second coolant (OrbisC™). This data was collected from a "PSC-1" cooler as supplied by the applicant, when fitted with a single cooling cap 210 as illustrated in Fig. 1. As can be seen, the flow rate of the second coolant (OrbisC™) is over twice that of the first coolant (glycol) over the temperature range of -10 °C to 0 °C. Also, the flow rate of the second coolant (OrbisC™) does not vary as much with temperature (2 ml/s or 7 % between -10 °C and 0 °C) when compared with the first coolant (glycol) (5 ml/s or 38 % between -10 °C and 0 °C). It is clear from Fig. 6 that the flow rate of the first coolant (glycol) through the cooling cap is drastically affected by the operating temperature, whereas the flow rate of the second coolant (OrbisC™) suffers only slight changes in comparision.

Over two roughly 40 minute test periods, where the apparatus 10 was applied to the scalp of a test patient, the following heat extraction results were obtained.

For the first coolant (glycol) - 44 minutes and 32 seconds at a flow rate of 9 ml/s:

$m_1$ = 21.31 kg (volume = 24074 ml)

$c_1$ = 3860 J/kg·K

$\Delta T_1$ = 1.85 K (Bath set-point = -10 °C, T1 = -8.11 °C, T2 = -6.26 °C)

Applying Equation 1, the amount of heat energy transferred (E) during the 40 minute period using the first coolant (glycol) was 152.16 kJ.

For the second coolant (OrbisC™) - 47 minutes and 9 seconds at a flow rate of approximately 27 ml/s:

$m_1$ = 66.32 kg (volume = 76284 ml)

$c_1$ = 3380 J/kg·K

$\Delta T_1$ = 0.88 K (Bath set-point = -10 °C, T1 = -8.5°C, T2 = -7.62 °C)

Applying Equation 1, the amount of heat energy transferred (E) during the 40 minute period using the second coolant (OrbisC™) was 197.27 kJ.

The second coolant (OrbisC™) has removed approximately 30% more heat energy. This is mainly due to the increased volumes of coolant flowing through the cap 210, made possible in this example by the lower viscosity of the second coolant (OrbisC™).

The next step was to establish a bath set-point temperature for the second coolant (OrbisC™) that removes the same amount of energy as the first coolant (glycol) at a bath set-point temperature of -10 °C.

Experimental results with the same test patient, in which the bath set-point temperature was varied, showed that the bath set-point temperature for the second coolant should be set at -2 °C to achieve approximately 152 kJ of heat energy removed. See Fig. 5.

Fig. 9 shows a graph of scalp energy removed at bath set-point temperatures ranging from -10 °C to -2 °C. As can be seen by the overlaid straight line, there is a close linear relationship measured in practice between the bath set-point temperature and the energy removed, using the second coolant (OrbisC™). The straight line is represented by the linear equation:

$$E = -6.39 \times \text{Bath temperature} + 135 \quad \text{(Equation 2)}$$

Where:

$E$ = the heat energy removed in joules (J);

-6.39 and 135 are constants; and

bath temperature = the bath set-point temperature in degrees Celsius (°C).

Of course, this equation is dependent on the use of the Applicant's own equipment, in this example the PSC-1 modified to have inlet and outlet temperature sensors and a flow rate sensor. The PSC-1 will have a unique layout and build, but other equations may be derived for other scalp coolers or body part temperature regulators from empirical results.

To verify the results, the scalp surface temperature of the test patient was measured with respect to the coolant bath set-point temperature. The scalp surface temperature was measured by fixing a k-type thermocouple to the surface of the scalp under the hair. An insulating barrier was placed between the thermocouple and the cap in order to minimise the effect of the cap temperature on the data. The Applicant recognises that the scalp surface temperature is lower than the actual scalp temperature due to the influence of the cap.

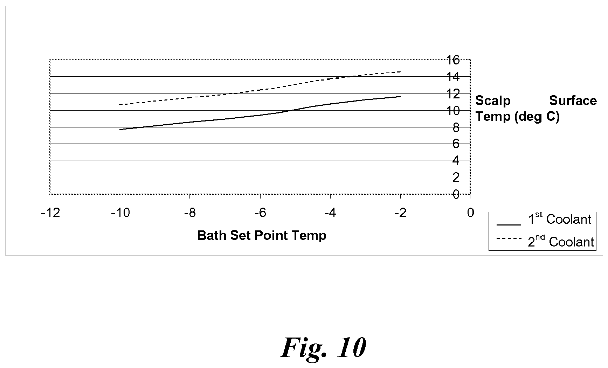

Fig. 10 shows a graph of the scalp surface temperatures measured using the first coolant (glycol) and the second coolant (OrbisC™) at various bath set-point temperatures. Knowing from experience that glycol used with a bath set-point temperature of -10 °C has positive results, this is used as the benchmark. The first coolant (glycol) set at -10 °C gave a scalp surface temperature of 10.6 °C, which is assumed to give a true scalp temperature of less that 20 °C, as required substantially to prevent hair loss.

From Fig. 10, we can see that the same scalp surface temperature of 10.6 °C can be achieved using the second coolant (OrbisC™) having a higher bath set-point temperature of -4 °C.

The results of the two practical experiments reveal that a lower bath set-point temperature for the second coolant (OrbisC™) can achieve the required scalp temperature. Equation 2 relating to heat energy extraction gives a target set-point of -2 °C, whereas measurement of the surface scalp temperature gives a lower target set-point of -4 °C. In any case, an increase in the bath set-point temperature of approximately 6 °C is feasible, which should lead to improved patient tolerance and higher success rates.

Fig. 10 reveals that the relationship between the bath set point temperature and the scalp surface temperature follows a good approximation to a linear trend, given below as:

$$\text{Bath set-point temperature} = 0.5 \times \text{Scalp surface temperature} + 12.6 \quad \text{(Equation 3)}$$

where the temperatures are measured in degrees Celsius (°C) and 0.5 and 12.6 are constants.

From Equation 3, which an equation for the scalp temperature can be derived using Equation 2, which is given below:

$$\text{Scalp surface temperature} = -0.079 \times \text{Energy Removed} + 23.33 \quad \text{(Equation 4)}$$

This equation, when compared with the results obtained experimentally, gives agreement to within 0.5 °C.

A direct relationship exists between the scalp surface temperature and the heat energy removed. Heat energy removed can easily be measured dynamically during treatment giving greater control potential. For example, it may be that during treatment too much heat energy is being removed which leads to the patient feeling uncomfortable. The controller 130 can adjust the bath set-point temperature gradually to reduce the heat energy removed to a level which is still acceptable for the treatment, but which does not make the patient unduly uncomfortable.

Further tests were carried out on 6 volunteer patients. Each patient underwent treatment using the first coolant (glycol at -10 °C) for 40 minutes, and using the second coolant (OrbisC™ at -4 °C) for 40 minutes. Heat extraction was recorded and comfort level was recorded. In all cases, more heat was extracted using the second coolant (OrbisC™) and the comfort level was better.

One patient experienced heat extraction of 199.57 kJ, and reported a low comfort level. Another patient experienced heat extraction of 145.91 kJ, and reported a high comfort level. With one minor exception, the amount of heat extracted directly correlated to the comfort level in an inverse relationship. That is, the more heat extracted, the less comfortable the patient.

From the previous experiments, it was seen that the two patients having the least amount of heat energy extracted would probably not have attained a scalp temperature to give the desired reduction in blood perfusion. Conversely, it appears that the patient experiencing the most heat energy extracted did so unnecessarily.

With real time monitoring of heat extracted, it is possible to adjust the coolant bath temperatures and/or flow rates to suit the patient. This could be done either automatically or with the intervention of the patient. For instance, when the apparatus 10 senses that either the heat extraction is too low or too high a control algorithm could be used or an alert could be outputted to allow the patient to make a single step adjustment to increase or decrease the heat extraction rate, respectively.

**CAP**

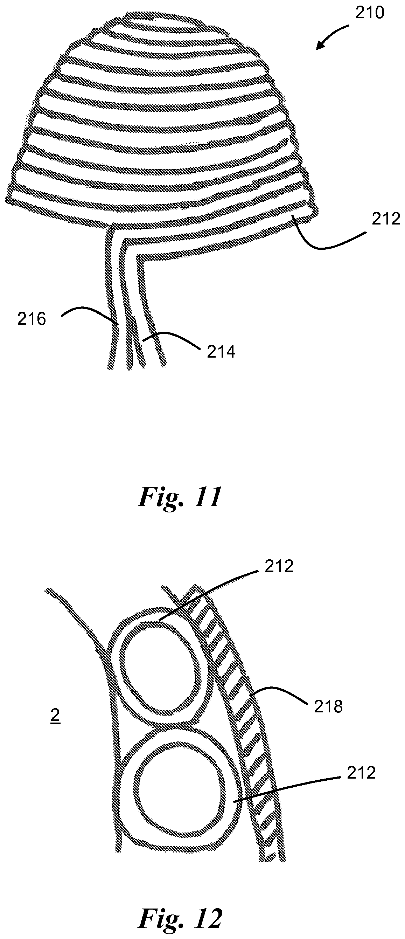

Fig. 11 shows the body part attachment, specifically the cap 210, of Fig. 1 in more detail.

The cap 210 comprises tubing 212 arranged in a continuous length and wound around in the approximate shape of a dome or a human scalp. The cap 210 comprises an inlet 214 and an outlet 216 which are arranged to be connected to flexible hosing 220 by quick release couplings (not shown). The cap 210 is suitable for placing over the human head and is semirigid.

Extra small (750 g), small (795 g), medium (810 g), large (850 g) and extra large (907 g) caps 210 are provided to fit the majority of patients' varying head sizes. The caps 210 are profiled to the contours of a typical head and are shaped around the ears, thereby increasing the level of comfort and acceptability for patients.

The tubing 212 is made from silicone rubber, comprising a polysiloxane backbone (silicon and oxygen), with hydrogen and carbon side groups. The silicone base material is then compounded, various other additives are incorporated and the resultant compound is then vulcanised using heat during manufacture of the tubing 212.

The tubing 212 in this example is manufactured from general purpose silicone rubber, which can be obtained commercially from Primasil Silicones Limited. The hardness of the tubing 212 is Shore A 40 and is available under product number PR110/40.

The inside diameter of the tubing 212 is 6 mm. The outside diameter of the tubing 212 is 8 mm. The wall thickness is 1 mm. Other sizes could be used.

In use, the tubing 212 is designed to have a wall which is thinner than existing tubing used in body part attachments 210, and in particular caps for cooling scalps during chemotherapy treatment. Also, the tubing 212 is designed to be softer, having a hardness rating of Shore A 40 as mentioned above. In combination, the reduced wall thickness and increased softness create tubing which expands under pressure to cause increased contact with the body part when compared with known body part attachments. In use, the apparatus 10 creates a pressure of approximately 110 kPa to 117 kPa (or 16 psi to 17 psi) in the tubing 212.

Fig. 12 is a partial cross-sectional view through the cap 212 of Fig. 10 when mounted on a patient's head 2 and having no coolant flow through the cap 212. The tubing 212 has a substantially circular cross section, although the tubing 212 is compressed slightly by the pressure exerted by an outer covering 218.

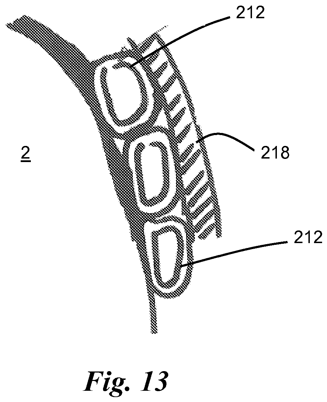

Fig. 13 is a partial cross-sectional view like Fig. 12, but in the case where coolant is flowing through the tubing 212 at approximately 110 kPa (16 psi). In this case, the outside diameter of the tubing 212, without the outside cover 218, would expand by approximately 20% to 9.6 mm. However, usefully, any expansion of between 5% and 50% would be advantageous, and most usefully between 15% and 25%.

Due to the outside cover 218, the tubing 212 flattens when expanding to fill in the gaps between the tubing and the patient's head 2. In this example, expansion of the outside diameter of the tubing 212 by 20% gives an approximate threefold increase in the surface area of the cap 210 in contact with the patient's head 2. However, the outside cover 218 is optional, as the resilience of the tubing 212 when wound as a cap 210 or similar, would cause the tubing 212 to compress against the patient's head 2 when the tubing 212 expands.

Usefully, the outside cover 218 covers the tubing 212 on the entire outside surface of the tubing 212. The outside cover 218 is rigid so as to direct the expansion of the tubes 212 to the inside surface of the cap 210, and hence to the patient's head in use. For this reason, the cover 218 is made from a resilient treated cotton material, but any other suitable material could be used, such as a plastics material. The idea is to direct the expansion of the tubing 212 against the body part to increase the surface area of the tubing 212 in contact with the body part. A chin-strap (not shown) is also included for this purpose.

The tubing 212, having an increased internal diameter of 6 mm when compared with other known tubing for this purpose, allows for an increased flow rate through the cap 210, which in turn helps to increase the maximum heat transfer rate of the apparatus 10. From the discussion above, it will be clear that this helps in the overall control of the treatment process, as more flexibility is introduced. For this same reason, the tubing 212 has a minimum curve radius of 5 mm.

**Summary**

A body part temperature regulating apparatus has been shown and described which is of general application in the field of body part temperature control, but which is particularly well suited to cooling to inhibit the effects of chemotherapy.

While the description focuses on scalp coolers, other body part could be cooled using the invention. Suitable adaptation may be required for fingers and toes, for example. This is because the human or animal body fights to protect core temperature at essential organs, such as the brain. In practice, this means that the temperature gradient between the brain at 37 °C and the coolant 190 needs to be at a first gradient to achieve the desired scalp temperature of less than 20 °C. Other gradients may be used for other body parts which are not as close to essential organs.

The invention provides an improved body part temperature regulating apparatus which is able to control more accurately the temperature of the body part in question by measuring the heat energy extracted from the body part. Experiments show that the temperature of the body part is correlated to the amount of heat energy extracted over time.

The apparatus is also more convenient and possibly more accurate than those apparatuses of the prior art which measure directly the temperature of the heat transfer fluid in the body part covering or the body part covering surface itself.

As a useful side effect, the apparatus is more energy efficient, extracting closer to the minimum necessary heat from the body part. Also, if the temperature of the heat transfer fluid can be raised, such as in circumstances in which a user does not have a full head of hair, and hence has less insulation between the scalp, and the scalp covering, for example, the apparatus is able to achieve a pre-set condition much faster, thereby reducing waiting time.

The apparatus is also more flexible in the way temperature can be controlled. For example, patients undergoing treatment on different drugs will be able to have different pre-programmed and desired heat transfer characteristics to suit each drug, respectively.

Also, an improved cap is provided which achieves better contact with the human head and which helps to maintain relatively high flow rates, resulting in better heat transfer rates between the patient and the coolant 190.

An improved coolant is also provided which achieves good heat transfer and improved and consistent flow rates.

Although a few preferred embodiments have been shown and described, it will be appreciated by those skilled in the art that various changes and modifications might be made without departing from the scope of the invention, as defined in the appended claims.

**Claims**

1. A body part cooling apparatus (10) for regulating the temperature of a part of the human or animal body, the body part cooling apparatus (10) comprising:

   a refrigeration and control unit (100); and
   a body part cooling attachment (200);
   wherein:

   the refrigeration and control unit (100) comprises:

   a heat exchanger (110),
   a pump (120),
   an outlet connection (184) and an inlet connection (182)
   all of which are connected together via interconnecting pipe work (170);

   a first temperature sensor (160), a second temperature sensor (140) and a heat transfer fluid flow rate sensor (150); and
   a controller (130) arranged to receive respective signals from the first temperature sensor (160), the second temperature sensor (140) and the flow rate sensor (150) and

   to control operation of the heat exchanger (110), the pump (120), or both the heat exchanger (110) and the pump (120);
   the body part cooling attachment (200) comprises :

   an attachment part (210) for attaching to the human or animal body, and
   hosing (220) which connects the attachment part (210) to the outlet connection (184) and the inlet connection (182) of the refrigeration and control unit (100); the heat transfer fluid is disposed in the heat exchanger (110), pump (120), interconnecting pipe work (170), hosing (220) and attachment part for circulation by the pump (120); and

the controller (130) is arranged :

to monitor the difference in temperature of the heat transfer fluid across at least the attachment part (210) of the body part cooling attachment and
to monitor the flow rate of the heat transfer fluid through the attachment part (210), and
to calculate the heat energy removed by the attachment part (210) over a predetermined period of time, and
to control the heat exchanger (110) or the pump (120), or both the heat exchanger (110) and the pump (120), to control the heat energy removed from the part of the human or animal body by the attachment part based on the calculated heat energy extracted,

**characterised in that**

the heat energy extracted follows a predetermined heat transfer characteristic,
the predetermined heat transfer characteristic is variable over time, and
the heat transfer characteristic is determined according to a drug, drug combination and/or drug dosage to be administered to the human or animal.

2. A body part temperature regulating apparatus (10) as claimed in claim 1, wherein the apparatus further comprises means to store the predetermined heat transfer characteristic.

3. A body part temperature regulating apparatus (10) according to claim 1 or claim 2 wherein the apparatus is configured to permit variation of the predetermined heat transfer characteristic.

4. A body part temperature regulating apparatus (10) as claimed in claim 1, claim 2 or claim 3,
wherein the controller (130) is arranged
to determine the amount of heat transferred to the heat transfer fluid using signals from the first temperature sensor (160), the second temperature sensor (140) and the flow rate sensor (150) during the first predetermined period of time and
to output a control signal to regulate the amount of heat transferred to the heat transfer fluid based on the determined amount of heat transferred so that the amount of heat transferred follows the predetermined heat transfer characteristic.

5. A body part temperature regulating apparatus (10) according to claim 4 wherein the controller (130) is arranged to determine the amount of heat transferred to the heat transfer fluid by:

(a) measuring the difference in temperature of the heat transfer fluid detected by the first temperature sensor (160) and the second temperature sensor (140) over the first predetermined period of time,
(b) determining the mass of the heat transfer fluid which has passed the temperature sensors over the first predetermined period of time from the flow rate detected by the flow sensor (150) and a predetermined value indicating the mass per unit volume of the heat transfer fluid; and
(c) using a predetermined value corresponding to the specific heat capacity of the heat transfer fluid.

6. A body part temperature regulating apparatus (10) according to any one of the preceding claims wherein the body part attachment (210) comprises
a length of tubing (212) and
the outside diameter of the tubing (212) is expandable to increase the surface area of the body part attachment (210) when in contact with the body part in use.

7. A body part temperature regulating apparatus (10) according to claim 6 wherein the body part attachment (210) is a cap for covering a scalp.

8. A body part temperature regulating apparatus (10) according to any preceding claim wherein the first temperature sensor (160) and the second temperature sensor (140) are located so as to measure the temperature difference across the body part attachment (210).

9. A body part temperature regulating apparatus (10) according to any preceding claim wherein the heat transfer fluid is an aqueous coolant composition comprising a freeze point depressant, a corrosion inhibitor and water.

**Patentansprüche**

1. Körperteilkühlvorrichtung (10) zum Regulieren der Temperatur eines Teils des menschlichen Körpers oder Tierkörpers, wobei die Körperteilkühlvorrichtung (10) umfasst:

    eine Kühl- und Steuereinheit (100); und
    eine Körperteilkühlbefestigung (200);
    wobei:

        die eine Kühl- und Steuereinheit (100) umfasst:

            einen Wärmetauscher (110),
            eine Pumpe (120),
            eine Auslassverbindung (184) und eine Einlassverbindung (182),
            die alle über Verbindungs-Rohrleitungen (170) miteinander verbunden sind;

        einen ersten Temperatursensor (160), einen zweiten Temperatursensor (140) und einen Wärmeübertragungsfluid-Flussratensensor (150); und
        eine Steuerung (130), die zum Empfangen entsprechender Signale von dem ersten Temperatursensor (160), dem zweiten Temperatursensor (140) und dem Flussratensensor (150) und zum Steuern des Betriebs des Wärmetauschers (110), der Pumpe (120) oder sowohl des Wärmetauschers (110) als auch der Pumpe (120) gestaltet ist;

        wobei die Körperteilkühlbefestigung (200) umfasst:

            einen Befestigungsteil (210) zum Befestigen an dem menschlichen Körper oder Tierkörper und
            Schläuche (220), die den Befestigungsteil (210) mit der Auslassverbindung (184) und der Einlassverbindung (182) der Kühl- und Steuereinheit (100) verbinden;

        wobei das Wärmeübertragungsfluid in dem Wärmetauscher (110), der Pumpe (120), den Verbindungs-Rohrleitungen (170), den Schläuchen (220) und dem Befestigungsteil zum Umwälzen durch die Pumpe (120) angeordnet ist; und
        die Steuerung (130) dafür gestaltet ist:

            den Unterschied der Temperatur des Wärmeübertragungsfluids über wenigstens den Befestigungsteil (210) der Körperteilkühlbefestigung zu überwachen und die Flussrate des Wärmeübertragungsfluids durch den Befestigungsteil (210) zu überwachen und
            die Wärmeenergie, die von dem Befestigungsteil (210) über eine vorbestimmte Zeitspanne entnommen wird, zu berechnen und
            den Wärmetauscher (110) oder die Pumpe (120) oder sowohl den Wärmetauscher (110) als auch die Pumpe (120) zu steuern, um die Wärmeenergie, die von dem Befestigungsteil aus dem Teil des menschlichen Körpers oder Tierkörpers entnommen wird, auf der Grundlage der berechneten entnommenen Wärmeenergie zu steuern,

    **dadurch gekennzeichnet, dass**

        die entnommene Wärmeenergie einer vorbestimmten Wärmeübertragungscharakteristik folgt,
        die vorbestimmte Wärmeübertragungscharakteristik im Zeitverlauf variabel ist und
        die Wärmeübertragungscharakteristik einem Arzneimittel, einer Arzneimittelkombination und/oder Arzneimitteldosierung, die an den Menschen oder das Tier verabreicht werden soll, entsprechend berechnet wird.

2. Körperteiltemperaturreguliervorrichtung (10) gemäß Anspruch 1, wobei die Vorrichtung ferner Einrichtungen zum Speichern der vorbestimmten Wärmeübertragungscharakteristik umfasst.

3. Körperteiltemperaturreguliervorrichtung (10) gemäß Anspruch 1 oder Anspruch 2, wobei die Vorrichtung dafür gestaltet ist, Variation der vorbestimmten Wärmeübertragungscharakteristik zu erlauben.

4. Körperteiltemperaturreguliervorrichtung (10) gemäß Anspruch 1, Anspruch 2 oder Anspruch 3,

wobei die Steuerung (130) dafür gestaltet ist,

die Menge der Wärme, die zu dem Wärmeübertragungsfluid übertragen wird, unter Verwendung von Signalen von dem ersten Temperatursensor (160), dem zweiten Temperatursensor (140) und dem Flussratensensor (150) während der ersten vorbestimmten Zeitspanne zu bestimmen und

ein Steuersignal auszugeben, um die Menge an Wärme, die zu dem Wärmeübertragungsfluid übertragen wird, auf der Grundlage der bestimmten Menge an übertragener Wärme zu regulieren, so dass die Menge an übertragener Wärme der vorbestimmten Wärmeübertragungscharakteristik folgt.

5. Körperteiltemperaturreguliervorrichtung (10) gemäß Anspruch 4, wobei die Steuerung (130) dafür gestaltet ist, die Menge an Wärme, die zu dem Wärmeübertragungsfluid übertragen wird, zu bestimmen durch:

(a) Messen des Unterschieds der von dem ersten Temperatursensor (160) und dem zweiten Temperatursensor (140) erfassten Temperatur des Wärmeübertragungsfluids über die erste vorbestimmte Zeitspanne,
(b) Bestimmen der Masse des Wärmeübertragungsfluids, das während der ersten vorbestimmten Zeitspanne an den Temperatursensoren vorbeigetreten ist, aus der von dem Durchflusssensor (150) erfassten Flussrate und einem vorbestimmten Wert, der die Masse pro Volumeneinheit des Wärmeübertragungsfluids angibt; und
(c) Verwenden eines vorbestimmten Werts, der der spezifischen Wärmekapazität des Wärmeübertragungsfluids entspricht.

6. Körperteiltemperaturreguliervorrichtung (10) gemäß einem der vorstehenden Ansprüche, wobei die Körperteilbefestigung (210) umfasst

einen Schlauchleitungsabschnitt (212) und
wobei der Außendurchmesser der Schlauchleitung (212) expandierbar ist, um die Oberfläche der Körperteilbefestigung (210) zu erhöhen, wenn sie in Verwendung mit dem Körperteil in Kontakt steht.

7. Körperteiltemperaturreguliervorrichtung (10) gemäß Anspruch 6, wobei die Körperteilbefestigung (210) eine Kappe zum Bedecken von Kopfhaut ist.

8. Körperteiltemperaturreguliervorrichtung (10) gemäß einem der vorstehenden Ansprüche, wobei der erste Temperatursensor (160) und der zweite Temperatursensor (140) angeordnet sind, um den Temperaturunterschied über die Körperteilbefestigung (210) zu messen.

9. Körperteiltemperaturreguliervorrichtung (10) gemäß einem der vorstehenden Ansprüche, wobei das Wärmeübertragungsfluid eine wässrige Kühlmittelzusammensetzung ist, die einen Gefrierpunktsenker, einen Korrosionsheller und Wasser umfasst.

**Revendications**

1. Appareil (10) de refroidissement d'une partie de l'organisme permettant de réguler la température d'une partie de l'organisme humain ou animal, l'appareil (10) de refroidissement d'une partie de l'organisme comprenant :

une unité de réfrigération et de commande (100) ; et
un accessoire (200) de refroidissement d'une partie de l'organisme ;
dans lequel :

l'unité de réfrigération et de commande (100) comprend :

un échangeur thermique (110),
une pompe (120),
un raccord de sortie (184) et un raccord d'entrée (182)
tous ces éléments étant raccordés ensemble par l'intermédiaire d'un montage de tuyaux d'interconnexion (170) ;

un premier capteur de température (160), un second capteur de température (140) et un capteur de débit (150) de fluide caloporteur ; et

un organe de commande (130) conçu pour recevoir des signaux respectifs en provenance du premier capteur de température (160), du second capteur de température (140) et du capteur de débit (150) et pour commander le fonctionnement de l'échangeur thermique (110), de la pompe (120), ou à la fois de l'échangeur thermique (110) et de la pompe (120) ;

l'accessoire (200) de refroidissement d'une partie de l'organisme comprend :

une partie accessoire (210) permettant une fixation à l'organisme humain ou animal, et
un tuyau (220) qui relie la partie accessoire (210) au raccord de sortie (184) et au raccord d'entrée (182) de l'unité de réfrigération et de commande (100) ;

le fluide caloporteur est disposé dans l'échangeur thermique (110), la pompe (120), le montage de tuyaux d'interconnexion (170), le tuyau (220) et la partie accessoire pour la circulation par la pompe (120) ; et
l'organe de commande (130) est conçu :

pour surveiller la différence de température du fluide caloporteur sur au moins la partie accessoire (210) de l'accessoire de refroidissement de partie de l'organisme et
pour surveiller le débit du fluide caloporteur à travers la partie accessoire (210), et
pour calculer l'énergie thermique éliminée par la partie accessoire (210) sur une période de temps prédéfinie, et
pour commander l'échangeur thermique (110) ou la pompe (120), ou à la fois l'échangeur thermique (110) et la pompe (120), pour réguler l'énergie éliminée de la partie de l'organisme humain ou animal par la partie accessoire sur la base de l'énergie thermique calculée extraite,
**caractérisé en ce que**

l'énergie thermique extraite suit une caractéristique de transfert thermique prédéfinie,
la caractéristique de transfert thermique prédéfinie varie dans le temps, et
la caractéristique de transfert thermique est déterminée en fonction d'un médicament, d'une association médicamenteuse et/ou d'un dosage de médicament à administrer à l'humain ou à l'animal.

2. Appareil (10) de régulation de la température d'une partie de l'organisme selon la revendication 1, l'appareil comprenant en outre des moyens pour enregistrer la caractéristique de transfert thermique prédéfinie.

3. Appareil (10) de régulation de la température d'une partie de l'organisme selon la revendication 1 ou la revendication 2, l'appareil étant conçu pour permettre une variation de la caractéristique de transfert thermique prédéfinie.

4. Appareil (10) de régulation de la température d'une partie de l'organisme selon la revendication 1, la revendication 2 ou la revendication 3,
dans lequel l'organe de commande (130) est conçu
pour déterminer la quantité de chaleur transférée vers le fluide caloporteur à l'aide de signaux provenant du premier capteur de température (160), du second capteur de température (140) et du capteur de débit (150) pendant la première période de temps prédéfinie et
pour sortir un signal de commande afin de réguler la quantité de chaleur transférée vers le fluide caloporteur sur la base de la quantité déterminée de chaleur transférée de sorte que la quantité de chaleur transférée suive la caractéristique de transfert thermique prédéfinie.

5. Appareil (10) de régulation de la température d'une partie de l'organisme selon la revendication 4 dans lequel l'organe de commande (130) est conçu pour déterminer la quantité de chaleur transférée vers le fluide caloporteur en :

(a) mesurant la différence de température du fluide caloporteur détectée par le premier capteur de température (160) et le second capteur de température (140) pendant la première période de temps prédéfinie,
(b) déterminant la masse du fluide caloporteur qui est passée par les capteurs de température pendant la première période de temps prédéfinie à partir du débit détecté par le capteur de débit (150) et d'une valeur prédéfinie indiquant la masse par unité de volume du fluide caloporteur ; et
(c) utilisant une valeur prédéfinie correspondant à la capacité thermique spécifique du fluide caloporteur.

6. Appareil (10) de régulation de la température d'une partie de l'organisme selon l'une quelconque des revendications précédentes dans lequel l'accessoire (210) de partie de l'organisme comprend

une longueur donnée de tube (212) et

le diamètre externe du tube (212) est déployable pour augmenter la surface spécifique de l'accessoire (210) de partie de l'organisme lors d'un contact avec la partie de l'organisme pendant l'utilisation.

7. Appareil (10) de régulation de la température d'une partie de l'organisme selon la revendication 6 dans lequel l'accessoire (210) de partie de l'organisme est une coiffe pour couvrir un cuir chevelu.

8. Appareil (10) de régulation de la température d'une partie de l'organisme selon l'une quelconque des revendications précédentes dans lequel le premier capteur de température (160) et le second capteur de température (140) sont situés de façon à mesurer la différence de température sur l'ensemble de l'accessoire (210) de partie de l'organisme.

9. Appareil (10) de régulation de la température d'une partie de l'organisme selon l'une quelconque des revendications précédentes dans lequel le fluide caloporteur est une composition réfrigérante aqueuse comprenant un dépresseur de point de congélation, un inhibiteur de la corrosion et de l'eau.

10

220

210

200

100

139

101

101

*Fig. 1*

*Fig. 2*

*Fig. 3*

*Fig. 4*

START

CONNECT BODY PART COOLING ATTACHMENT — S500

PERFORM PURGE — S510

PERFORM PRE-OPERATION COOLING — S520

APPARATUS READY FOR USE — S530

STOP

*Fig. 5*

*Fig. 6*

```
                    ┌─────────────┐
                    │    START    │
                    └─────────────┘
                           │
                           ▼
        ┌──────────────────────────────┐
        │  MEASURE FLOW RATE THROUGH    │
        │  BODY PART ATTACHMENT OVER    │      S700
        │  PREDETERMINED PERIOD OF      │
        │           TIME                │
        └──────────────────────────────┘
                           │
                           ▼
        ┌──────────────────────────────┐
        │    MEASURE TEMPERATURE        │
        │  DIFFERENCE (ΔT) ACROSS BODY  │
        │  PART ATTACHMENT OVER THE     │      S710
        │  PREDETERMINED PERIOD OF      │
        │           TIME                │
        └──────────────────────────────┘
                           │
                           ▼
        ┌──────────────────────────────┐
        │   CALCULATE MASS (m) FROM     │      S720
        │    FLOW RATE AND DENSITY      │
        └──────────────────────────────┘
                           │
                           ▼
        ┌──────────────────────────────┐
        │  CALCULATE HEAT TRANSFER (E)  │
        │   OVER THE PREDETERMINED      │      S730
        │  PERIOD OF TIME (E = c · m · ΔT) │
        └──────────────────────────────┘
                           │
                           ▼
        ┌──────────────────────────────┐
        │  OUTPUT TEMPERATURE CONTOL    │
        │   SIGNAL OR PUMP CONTROL      │      S740
        │  SIGNAL TO REGULATE HEAT      │
        │        TRANSFER RATE          │
        └──────────────────────────────┘
                           │
                           ▼
                    ┌─────────────┐
                    │    STOP     │
                    └─────────────┘
```

MEASURE FLOW RATE THROUGH BODY PART ATTACHMENT OVER PREDETERMINED PERIOD OF TIME — S700

MEASURE TEMPERATURE DIFFERENCE ($\Delta T$) ACROSS BODY PART ATTACHMENT OVER THE PREDETERMINED PERIOD OF TIME — S710

CALCULATE MASS ($m$) FROM FLOW RATE AND DENSITY — S720

CALCULATE HEAT TRANSFER ($E$) OVER THE PREDETERMINED PERIOD OF TIME ($E = c \cdot m \cdot \Delta T$) — S730

OUTPUT TEMPERATURE CONTOL SIGNAL OR PUMP CONTROL SIGNAL TO REGULATE HEAT TRANSFER RATE — S740

*Fig. 7*

*Fig. 8*

*Fig. 9*

**Fig. 10**

**Fig. 11**

**Fig. 12**

*Fig. 13*

**EP 2 603 183 B1**

**Patent documents cited in the description**

- US 4566455 A, Kramer
- WO 9816176 A, Olofsson
- GB 2323915 A
- WO 2006126059 A
- US 5509801 A, Nicholson
- US 2004210283 A1, Rose Joseph Lorney
- WO 9944552 A1
- US 2008077202 A1, Levinson Mitchell